# EUROPÄISCHE PATENTSCHRIFT

(11) **EP 1 200 384 B1**
(45) Veröffentlichungstag und Bekanntmachung des Hinweises auf die Patenterteilung: **28.04.2004**
(21) Anmeldenummer: 00940109.2
(22) Anmeldetag: 13.07.2000
(51) Int. Cl.: C07C 69/65, C07C 57/52, C07C 57/76, C07C 51/06, C07D 263/26

(54) **2-ALKYL-5-HALOGEN-PENT-4-ENCARBONSÄUREN UND DEREN HERSTELLUNG**
2-ALKYL-5-HALOGEN-PENT-4-ENE CARBOXYLIC ACIDS AND THEIR PRODUCTION
ACIDES ALKYLE-5-HALOGENE-PENT-4-ENE-CARBOXYLIQUES ET LEUR PREPARATION

(30) Priorität: 29.07.1999 CH 140199; 11.01.2000 CH 44002000
(43) Veröffentlichungstag der Anmeldung: 02.05.2002
(73) Patentinhaber: Speedel Pharma AG, 4051 Basel (CH)
(72) Erfinder: HEROLD, Peter, CH-4057 Basel (CH); STUTZ, Stefan, CH-4053 Basel (CH)
(74) Vertreter: Dannappel, Hans-Jochen, Dr.
(86) Internationale Anmeldenummer: PCT/CH2000/000385
(87) Internationale Veröffentlichungsnummer: WO 2001/009079

(56) Entgegenhaltungen:
- US-A- 4 492 799
- CHEMICAL ABSTRACTS, vol. 55, no. 21, 16. Oktober 1961 (1961-10-16) Columbus, Ohio, US; abstract no. 20950h, M. T. DANGYAN ET AL.: "Preparation of Alkyl-gamma-chlorallylacetic Acids" Seite 20950; Spalte 2; XP002149121 & IZVEST. AKAD. NAUK ARMYAN. S.S.R., KHIM. NAUKI, Bd. 13, Nr. 4, 1960, Seiten 259-262,
- CHEMICAL ABSTRACTS, vol. 70, no. 23, 9. Juni 1969 (1969-06-09) Columbus, Ohio, US; abstract no. 105904w, L. A. SAAKYAN ET AL.: "Molecular Rearrangements. XII. Synthesis and Transformations of 3-Substituted 6,6-Dichloro-5-hexenoic Acids " Seite 274; Spalte 2; XP002149122 & ARM. KHIM. ZH., Bd. 21, Nr. 11, 1968, Seiten 975-980,

## Beschreibung

Die Erfindung betrifft 2-Alkyl-5-Halogen-pent-4-encarbonsäure sowie dessen Ester und Salze in Form der Racemate und Enantiomeren, und ein Verfahren zur Herstellung dieser Carbonsäuren.

In der EP-A-0 678 503 werden d-Amino-g-hydroxy-w-aryl-alkancarbonsäureamide beschrieben, die Reninhemmende Eigenschaften aufweisen und als antihypertensive Mittel in pharmazeutischen Zubereitungen verwendet werden können. Die beschriebenen Herstellungsverfahren sind hinsichtlich der Anzahl von Verfahrensstufen und Ausbeuten unbefriedigend und nicht für ein industrielles Verfahren geeignet. Nachteilig bei diesen Verfahren ist auch, dass reine Diastereomere in zu geringen Gesamtausbeuten erhältlich sind.

In einem neuen Verfahren geht man von 2,7-Dialkyl-8-aryl-4-octenoylamiden aus, dessen Doppelbindung gleichzeitig in 5-Stellung halogeniert und in 4-Stellung unter Lactonisierung hydroxyliert wird, dann das Halogen mit Azid ersetzt, das Lacton amidiert und darauf das Azid in die Amingruppe überführt wird. Die gewünschten Alkancarbonsäureamide werden beim neuen Verfahren sowohl in hohen Gesamtausbeuten als auch in hoher Reinheit erhalten, und es können gezielt reine Diastereomere hergestellt werden. Die Halolaktonisierung der Verfahrensstufe a), die Azidierung der Verfahrensstufe b) und die Azidreduktion der Verfahrensstufe d) sind von P. Herold im Journal of Organic Chemistry, Vol. 54 (1989), Seiten 1178-1185 beschrieben.

Die 2,7-Dialkyl-8-aryl-4-octenoylamide können zum Beispiel der Formel A entsprechen, 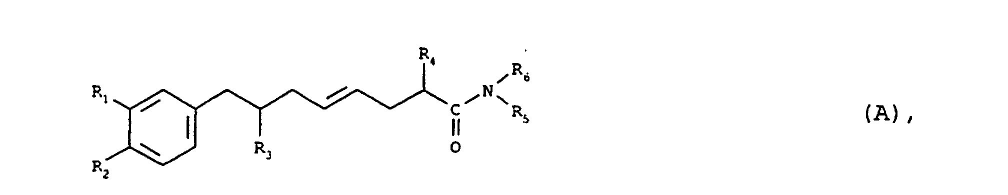 und insbesondere der Formel A1 entsprechen 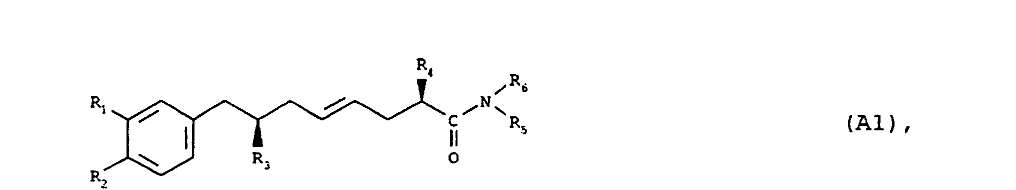 worin R₁ und R₂ unabhängig voneinander H, C₁-C₆-Alkyl, C₁-C₆-Halogenalkyl, C₁-C₆-Alkoxy, C₁-C₆-Alkoxy-C₁-C₆-Alkyl, oder C₁-C₆-Alkoxy-C₁-C₆-Alkyloxy darstellen, R₃ C₁-C₆-Alkyl bedeutet, R₄ für C₁-C₆-Alkyl steht, R₆ C₁-C₆-Alkyl darstellt, R₅ C₁-C₆-Alkyl oder C₁-C₆-Alkoxy bedeutet, oder R₅ und R₆ zusammen gegebenenfalls mit C₁-C₄-Alkyl, Phenyl oder Benzyl substituiertes Tetramethylen, Pentamethylen, 3-Oxa-1,5-Pentylen oder -CH₂CH₂O-C(O)- sind.

Die Verbindungen der Formel A und A1 sind erhältlich, indem man eine Verbindung der Formel B 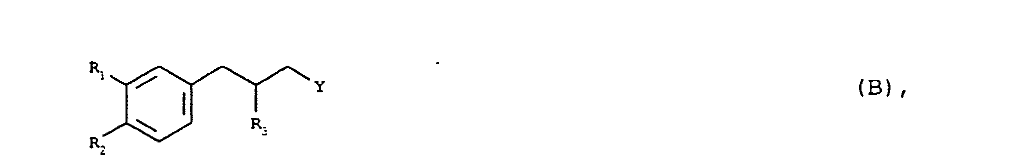 als Racemat oder Enantiomere, mit einer Verbindung der Formel C, als Racemat oder Enantiomere, 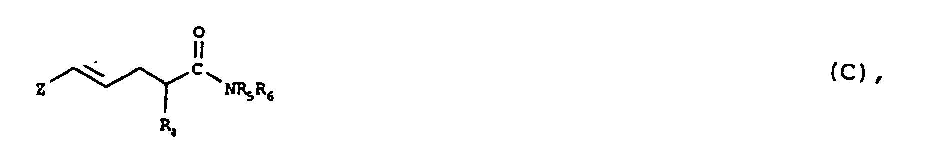 worin R₁ bis R₄, R₅ und R₆ die zuvor angegebenen Bedeutungen haben, Y Cl, Br oder I und Z Cl, Br oder I bedeuten, in Gegenwart eines Alkali- oder Erdalkalimetalls umsetzt. Y und Z bedeuten bevorzugt Br und besonders bevorzugt Cl.

Die Verbindungen der Formel C werden zweckmässig aus den den Amiden entsprechenden Carbonsäuren und deren Estern oder Säurehalogeniden hergestellt, die somit wertvolle Zwischenprodukte für die Herstellung der eingangs erwähnten antihypertensiven Mittel darstellen. Die Bildung von Carbonsäureamiden aus Carbonsäureestern und Aminen in Gegenwart von Trialkylaluminium oder Dialkylaluminiumhalogenid, zum Beispiel mit Trimethylaluminium oder Dimethylaluminiumchlorid, ist von S. M. Weinreb in Org. Synthesis, VI, Seite 49 (1988) beschrieben.

Ein Gegenstand der Erfindung sind Verbindungen der Formel Ia in Form der Racemate und Enantiomeren, 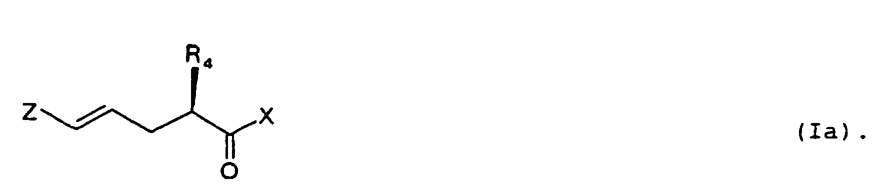 worin
R₄ für C₁-C₆-Alkyl steht, Z Chlor, Brom oder Iod bedeutet, und X -OH, Chlorid, Bromid oder Iodid darstellt, oder X mit dem Carbonylrest eine Estergruppe bildet, sowie Salze der Carbonsäuren.

R₄ steht bevorzugt für C₁-C₄-Alkyl. Beispiele für Alkyl sind Methyl, Ethyl, n- und i-Propyl, n-, i- und t-Butyl, Pentyl und Hexyl. Ganz besonders bevorzugt stellt R₄ i-Propyl dar.

Z steht besonders bevorzugt für Cl.

In der Estergruppe stellt X bevorzugt einen Rest der Formel R₇O- dar, worin R₇ eine gegebenenfalls Heteroatome, ausgewählt aus der Gruppe O und N, enthaltende organische Gruppe mit 1 bis 18, bevorzugt 1 bis 12, und besonders bevorzugt 1 bis 8 C-Atomen bedeutet.

Bei R₇ kann es sich um verzweigtes und vorzugsweise lineares Alkyl handeln, das bevorzugt 1 bis 4 C-Atome enthält. Beispiele sind Methyl, Ethyl, n-Propyl, n-Butyl, Pentyl, Hexyl, Heptyl und Octyl. Besonders bevorzugt sind Methyl und Ethyl. Das Alkyl kann substiuiert sein, zum Beispiel mit C₁-C₄-Alkoxy, wie Methoxy oder Ethoxy. Beispiele für substituiertes Alkyl sind Methoxyethyl und Ethoxyethyl.

Bei R₇ kann es sich um Cycloalkyl mit 3 bis 8, und bevorzugt 5 oder 6 Ringkohlenstoffatomen handeln. Beispiele sind Cyclopropyl, Cyclobutyl, Cyclohexyl, Cyclopentyl, Cyclohexyl, Cycloheptyl und Cyclooctyl. Das Cycloalkyl kann mit C₁-C₄-Alkyl oder C₁-C₄-Alkoxy substituiert sein.

Bei R₇ kann es sich um Cycloalkyl-C₁-C₄-alkyl mit 3 bis 8, und bevorzugt 5 oder 6 Ringkohlenstoffatomen handeln, das unsubstituiert oder mit C₁-C₄-Alkyl oder C₁-C₄-Alkoxy substituiert ist. Beispiele sind Cyclopentylmethyl, Cyclohexylmethyl, Methylcyclohexylmethyl und Cyclohexylethyl.

Bei R₇ kann es sich um C₆-C₁₀-Aryl handeln, das unsubstituiert oder mit C₁-C₄-Alkyl oder C₁-C₄-Alkoxy substituiert ist. Beispiele sind Phenyl, Naphthyl, Methylphenyl, Ethylphenyl und i-Propylphenyl.

Bei R₇ kann es sich um C₆-C₁₀-Aryl-C₁-C₄-alkyl handeln, das unsubstituiert oder mit C₁-C₄-Alkyl oder C₁-C₄-Alkoxy substituiert ist. Beispiele sind Benzyl, Methylbenzyl und Phenylethyl.

Unter den Säurehalogeniden der Formel Ia sind die Chloride und Bromide bevorzugt.

Bei den Salzen der Carbonsäuren kann es sich zum Beispiel um Alkalimetall- oder Erdalkalimetallsalze, sowie um Ammoniumsalze handeln. Unter den Alkalimetallen und Erdalkalimetallen sind Lithium, Natrium, Kalium, Magnesium und Calcium bevorzugt. Unter Ammonium ist das Ammoniumkation, die Kationen primärer, sekundärer und tertiärer Amine und quaternäres Ammonium zu nennen; diese Kationen können 1 bis 20, bevorzugt 1 bis 16 C-Atome enthalten.

Besonders bevorzugte Verbindungen der Formel Ia sind solche, worin Z Chlor bedeutet, R₄ C₁-C₄-Alkyl und besonders bevorzugt i-Propyl darstellt, und X für OH, Cl, Br oder C₁-C₄-Alkoxy steht.

Von einer ganz bevorzugten Ausführungsform werden Verbindungen der Formel Ia umfasst, worin Z Chlor bedeutet, R₄ i-Propyl darstellt, und X für OH, Cl, Br, Methoxy oder Ethoxy steht.

Insbesondere bevorzugt sind Verbindungen der Formel Ia, worin Z Chlor bedeutet, R₄ i-Propyl darstellt, und X für Cl oder Ethoxy steht.

Die Verbindungen der Formel Ia sind erhältlich, indem man zum Beispiel Isovaleriansäureester mit 1,3-Dihalogenpropen in Gegenwart von starken Aminbasen wie zum Alkalimetallamiden (Li-N(i-Propyl)₂ oder Lithiumhexamethyldisilazan) zu den Estern der Formel Ia in Form der Racemate umsetzt, und daraus durch Derivatisierung in an sich bekannter Weise die Carbonsäuren, die Carbonsäurehalogenide und Carbonsäuresalze herstellt. Aus den Racematen können in an sich bekannter Weise durch Racematspaltung die gewünschten Enantiomeren erhalten werden, zum Beispiel mittels Kristallisationsverfahren von Additionssalzen der Carbonsäuren mit optisch aktiven Basen. Vorteilhafter ist eine Racematspaltung durch Behandlung von Estern der Formel Ia in Form der Racemate mit Esterasen.

Ein Verfahren zur Herstellung der Racemate ist dadurch gekennzeichnet, dass man eine Verbindung der Formel II, 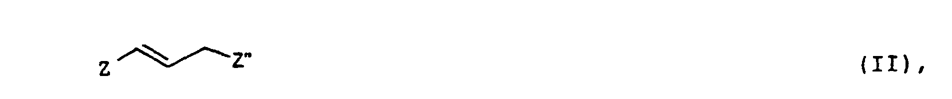 worin Z und Z" unabhängig voneinander Chlor, Brom oder Iod, und bevorzugt Chlor oder Brom darstellen, in Gegenwart von starken Aminbasen mit einer Verbindung der Formel III umsetzt, 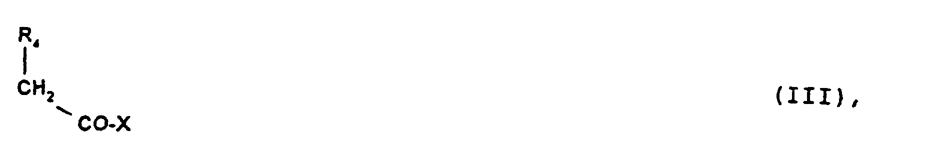 worin X mit dem Carbonylrest eine Estergruppe bildet und R₄ für C₁-C₆-Alkyl steht, und die erhaltenen Carbonsäureester der Formel Ia in Form der Racemate gegebenenfalls zu Carbonsäuren, Carbonsäurehalogeniden oder Carbonsäuresalzen derivatisiert.

Bei den starken Aminbasen handelt es sich vorzugsweise um Alkalimetallamide. Die Reaktion wird zweckmässig in Ethern als Lösungsmittel und unter Kühlung bis etwa Raumtemperatur durchgeführt. Kühlung kann bis etwa -20 °C bedeuten.

Die Verbindungen der Formel Ia können auch durch asymmetrische Synthese erhalten werden.

Ein weiterer Gegenstand der Erfindung ist ein Verfahren zur Herstellung von Verbindungen der Formel Ia, das dadurch gekennzeichnet ist, dass man. eine Verbindung der Formel IV, 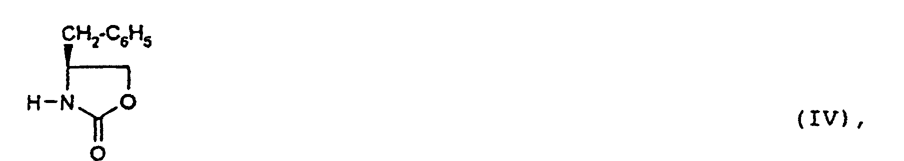 mit einem Carbonsäurehalogenid der Formel R₄CH₂-CO-X umsetzt, worin R₄ die zuvor angegebene Bedeutung hat und X für Chlor, Brom oder Iod steht, die erhaltene Verbindung der Formel V, 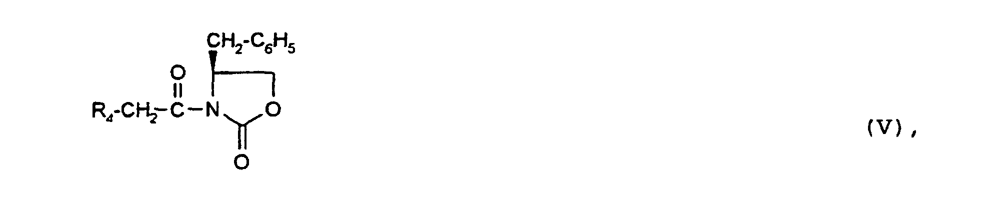 zuerst mit Lithiumhexamethyldisilazid und dann mit einer Verbindung der Formel II umsetzt, 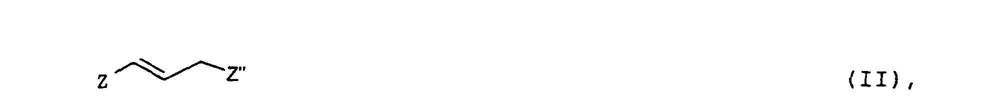 worin Z und Z" unabhängig voneinander Chlor, Brom oder Iod darstellen, danach die erhaltene Verbindung der Formel VI 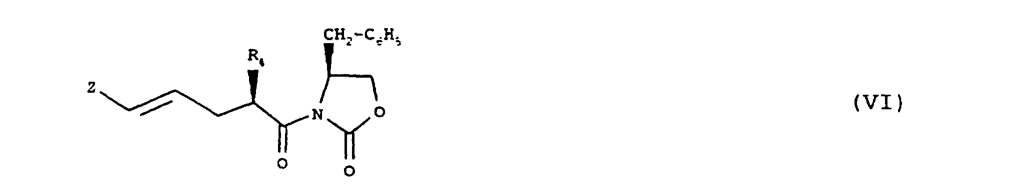 mit einer Base hydrolysiert, die Salze oder Carbonsäuren der Formel Ia isoliert, und gegebenenfalls die Carbonsäuren zu Estern oder Halogeniden derivatisiert.

Als Base werden vorzugsweise Alkalimetallbasen, zum Beispiel LiOH, NaOH oder KOH verwendet. Zur Beschleunigung der Hydrolyse können zusätzlich Oxidationsmittel verwendet werden, zum Beispiel Wasserstoffperoxid. Bei den einzelnen Verfahrensstufen handelt es sich um analoge Verfahren, die dem Fachmann geläufig sind und in den nachfolgenden Beispielen näher erläutert sind.

### A) Herstellung von Racematen

### Beispiel A1:

Herstellung von

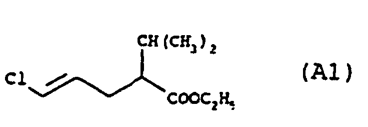

Eine gerührte Lösung von 77,7 ml Diisopropylamin und 200 ml Tetrahydrofuran wird auf -20 °C gekühlt und während 15 Minuten mit 200 ml 2,5 M n-Hexyllithiumlösung (in Hexan) versetzt. Die Lösung wird 15 Minuten bei -20 °C nachgerührt und anschliessend wird während 30 Minuten eine Lösung von 75,3 ml Isovaleriansäureethylester in 80 ml Tetrahydrofuran zugetropft. Die Lösung wird 10 Minuten nachgerührt und dann während 10 Minuten bei -20 °C mit 80 ml DMPU versetzt. Zugabe von 8,2 g Natriumiodid und 19,5 g trans-1,3-Dichlorpropen. Das Reaktionsgemisch wird noch 23 Stunden bei - 20 °C nachgerührt und anschliessend mit 500 ml 20%-iger wässriger Ammoniumchloridlösung versetzt. Die Mischung wird mit tert.-Butylmethylether (2x 400 ml) extrahiert und die organischen Phasen nacheinander mit 0,1 M Natriumthiosulfatlösung (1x 500 ml), Wasser (1x 500 ml) und Sole (1x 500 ml) gewaschen. Die vereinigten organischen Phasen werden mit 150 g Natriumsulfat getrocknet und eingedampft. Aus dem Rückstand wird mittels Destillation die Titelverbindung A1 als farbloses Oel erhalten (86,1 g, 84 %). ¹H-NMR (400 MHz, CDCl₃, δ) : 0,95 (m, 6H), 1,30 (t, 3H), 1,92 (m, 1H), 2,20 - 2,40 (m, 3H), 4,20 (m, 2H), 5, 80 - 6,10 (m, 2H) ppm.

### Beispiel A2:

Herstellung von

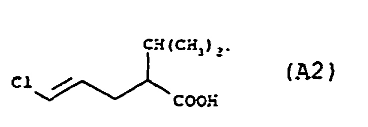

Eine Lösung von 150,2 g A1, 500 ml Ethanol und 500 ml 2N Natronlauge wird während 18 Stunden am Rückfluss gerührt. Aus dem Reaktionsgemisch wird das Ethanol abgedampft, die wässrige Lösung mit 1N Salzsäure angesäuert und mit Diethylether (3x) extrahiert. Die organischen Phasen werden mit Magnesiumsulfat getrocknet und eingedampft. Aus dem Rückstand wird mittels Flashchromatographie (SiO₂ 60F / Dichlormethan / Methanol 20:1) die Titelverbindung A2 als leicht oranges Oel erhalten (83,7 g, 65 %). ¹H-NMR (400 MHz, CDCl₃, δ) : 1,03 (m, 6H), 1,98 (m, 1H), 2,20 - 2,45 (m, 3H), 5,80 - 6,10 (m, 2H) ppm.

### B) Herstellung von Verbindungen der Formel Ia

### Beispiel B1:

Herstellung von

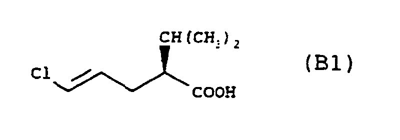

5,0 g A2, 5,0 g Cinchonidin und 1,98 ml Triethylamin werden in 150 ml Tetrahydrofuran vorgelegt und während 15 Minuten unter Rückfluss gerührt. Das Oelbad wird entfernt und die klare Lösung mit einem Salz von B1 mit Cinchonidin angeimpft. Man rührt während 1 Stunde bei Raumtemperatur und anschliessend noch 1 Stunde unter Eiskühlung. Der Niederschlag wird abfiltriert, mit 2 mal 25 ml eiskaltem Aceton gewaschen und anschliessend im Vakuum bei 50 °C bis zur Gewichtskonstanz getrocknet. Man erhält 6,16 g (46,3%) des angereicherten Salzes von B1 mit Cinchonidin; Schmelzpunkt 149 °C. Nach zweimaliger Umkristallisation aus Aceton erhält man 4,20 g (31,6%) des angereicherten Salzes von B1 mit Cinchonidin, Schmelzpunkt 155 °C. Das so erhaltene Salz wird zwischen 250 ml Diethylether und 50 ml 1N HCl verteilt. Die wässrige Phase wird abgetrennt, die organische Phase mit gesättigter NaCl-Lösung gewaschen, mit MgSO₄ getrocknet und im Vakuum eingedampft. Man erhält 1,58 g (31,6%) der angereicherten Verbindung B1 als farbloses Oel.

### Beispiel B2:

Herstellung von

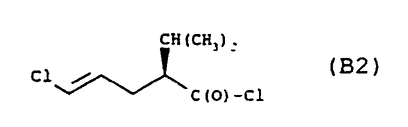

Eine Lösung von 4,54 g B1 in 25 ml Toluol wird bei Raumtemperatur mit 4,42 ml Oxalylchlorid versetzt. Das Reaktionsgemisch wird 15 Minuten bei Raumtemperatur gerührt und anschliessend während 1 Minute mit 0,052 ml N,N-Dimethylformamid versetzt. Das Reaktionsgemisch wird zum Rückfluss erwärmt und 1 Stunde gerührt. Die Reaktionslösung wird eingedampft und der Rückstand destilliert. Man erhält die Titelverbindung B2 als farbloses Oel: (4,43 g, 88 %). ¹H-NMR (400 MHz, CDCl₃, δ): 1,02 (d, 3H), 1,08 (d, 3H), 2,16 (m, 1H), 2,40 (m, 1H), 2,45 (m, 1H), 2,68 (m, 1H), 5,80 - 6,10 (m, 2H) ppm.

### Beispiel B3:

Herstellung von

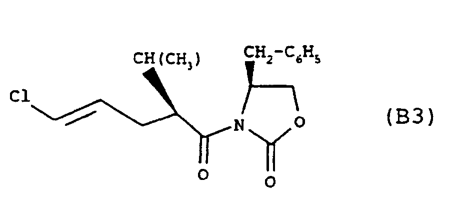

Eine Lösung von 290 g 4S-Benzyl-3-(3-methyl-butyryl)-oxazolidin-2-one in 0,58 l Tetrahydrofuran wird auf -78 °C gekühlt und während 65 Minuten 1,14 l 1 M Lithiumhexamethyldisilazid (in Tetrahydrofuran) zugetropft. Das Gemisch wird noch 1 Stunde bei -78 °C nachgerührt und anschliessend mit der vorbereiteten Lösung von trans-1-Chloro-3-iod-propen in Tetrahydrofuran versetzt. Man lässt die Temperatur auf 0 °C steigen und rührt noch weitere 20 Stunden nach. Das Reaktionsgemisch wird mit 500 ml 10%-iger Ammoniumchloridlösung versetzt und mit Diethylether extrahiert (2x 1 l). Die organischen Phasen werden mit Wasser (1x 1 l) gewaschen, mit Natriumsulfat getrocknet und eingedampft. Aus dem Rückstand wird mittels Flashchromatographie (SiO₂ 60F / Essigsäureethylester/Hexan 5:1) die Titelverbindung B3 als leicht oranges Oel erhalten (582 g, 78 %). ¹H-NMR (400 MHz, CDCl₃, δ) : 0,85 (m, 6H), 2,02(m, 1H), 2,3 - 2,55 (m, 2H), 2,75 (m, 1H), 3,30 (m, 1H), 3,88 (m, 1H), 4,18 (m, 2H), 4,70 (m, 1H), 5,80 - 6,10 (m, 2H), 7,15 - 7,40 (m, 5H) ppm. Herstellung von trans- 1-Chlor-3-iod-propen: Eine Lösung von 184,7 g trans-1,3-Dichlorpropen in 0,58 l Tetrahydrofuran wird mit 266,1 g Natriumiodid versetzt und unter Lichtausschluss während 30 Minuten bei Raumtemperatur gerührt. Das Gemisch wird klarfiltriert und das Filtrat direkt eingesetzt.

### Beispiel B4: Herstellung von B1

Zu einer bei 0° C gerührten Lösung von 155 g B3, 1,3 l Tetrahydrofuran und 0,44 l Wasser werden während 15 Minuten 315 ml 30%-ige Wasserstoffperoxidlösung zugetropft. Das Reaktionsgemisch wird mit 22,1 g Lithiumhydroxid versetzt, anschliessend das Kühlbad entfernt und dann 5 Stunden bei 0 - 20° C nachgerührt. Die Reaktionsmischung wird wiederum auf 0° C gekühlt und während 30 Minuten eine Lösung von 350 g Natriumsulfit in 1,4 l Wasser zugetropft. Durch Zugabe von Natriumhydrogencarbonat wird auf pH 9,8 gestellt. Das Reaktionsgemisch wird klarfiltriert und aus dem Filtrat das Tetrahydrofuran abgedampft. Die erhaltene wässrige Lösung wird mit Methylenchlorid (3x 3 l) gewaschen. Die Wasserphase wird mit wässriger Salzsäure auf pH 3,.0 gestellt und dann mit Methylenchlorid (3x 2l) extrahiert. Die organischen Phasen werden mit Magnesiumsulfat getrocknet und am Rotavapor eingedampft. Aus dem Rückstand wird mittels Destillation die Verbindung B1 als farbloses Oel erhalten. (142 g, 87 %). ¹H-NMR (400 MHz, CDCl₃, δ): 1, 02 (m, 6H , 1, 98 (m, 1H), 2,25 - 2,45 (m, 2H), 5,85 - 6,10 (m, 2H) ppm.

### Beispiel B5:

Herstellung von

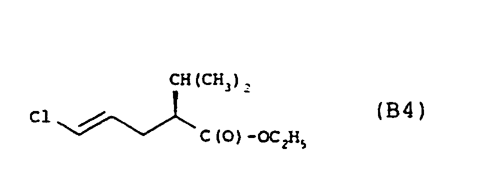

1 l Phosphatpuffer (pH 7,0) wird mit einer Lösung von 100 g A1 in 40 ml Isopropanol versetzt. In Gegenwart von 2,0 ml (4400 U) Schweineleberesterase. (Tech. Grade, Roche Diagnostics) wird bei pH 8,0 und 40° C bis zum Verbrauch von 262 ml 1,0 N NaOH gerührt. Das Reaktionsgemisch wird mit Ethylacetat ( 1x 1 l und 2x 0,5 l)) extrahiert. Die organischen Phasen werden nacheinander mit 5 %-iger wässriger Na₂CO₃-Lösung (3x 500 ml) und Sole (1x 0.5 l) gewaschen, mit 300 g Na₂SO₄ getrocknet, eingeengt und im Vakuum getrocknet. Aus dem Rückstand wird mittels Destillation die Titelverbindung B4 als farbloses Oel (45.4 g, 46 %) mit einem ee von grösser 99 % erhalten.

### Beispiel B6: Herstellung von (B1)

Eine Lösung von 150,2 g B4, 500 ml Ethanol und 500 ml 2N Natronlauge wird während 18 Stunden am Rückfluss gerührt. Aus dem Reaktionsgemisch wird das Ethanol abgedampft, die wässrige Lösung mit 1N Salzsäure angesäuert und mit Diethylether (3x) extrahiert. Die organischen Phasen werden mit Magnesiumsulfat getrocknet und eingedampft. Aus dem Rückstand wird mittels Flashchromatographie (SiO₂ 60F / Dichlormethan / Methanol 20:1) die Titelverbindung B1 als leicht gelbliches Oel (0,88 g, 43 %) mit einem ee von grösser 99 % erhalten.

### C) Anwendungsbeispiele

### Beispiel C1:

Herstellung von

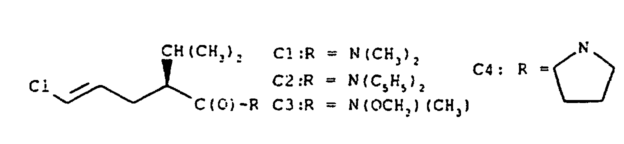

Eine Lösung von 1,53 g Dimethylamin, 3,66 ml Pyridin und 25 ml Dichlormethan wird auf 0 °C gekühlt und anschliessend 4,42 g B2 in 25 ml Dichlormethan bei 0 bis -10° C zugetropft. Das Reaktionsgemisch wird noch 2 Stunden bei 0 °C weitergerührt und anschliessend am Rotavapor eingedampft. Der Rückstand wird zwischen Diethylether (2x) und 2N Salzsäure (3x), gesättigter Natriumhydrogencarbonatlösung (1x) und gesättigter Kochsalzlösung verteilt. Die organischen Phasen werden vereinigt, über Natriumsulfat getrocknet und eingeengt. Der Rückstand wird destilliert und die Titelverbindung Cl als farbloses Oel erhalten: (4,13 g, 89 %). [α]²⁵ _{D} - 7,3 (*c* 1, Chloroform). ¹H-NMR (400 MHz, CDCl₃, δ): 0,90 (d, 3H), 0,95 (d, 3H), 1,92 (m, 1H), 2,20 - 2,30 (m, 1H), 2, 35 - 2,50 (m, 2H), 2,98 (s,3H), 3,04 (s, 3H), 5, 80 - 6,10 (m, 2H) ppm.

Analog Beispiel C1 werden aus dem Säurechlorid B2 und den entsprechenden Aminen, die Derivate C2, C3 und C4 hergestellt.

### Beispiel C2:

Herstellung von

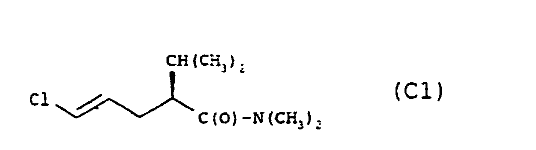

Eine Mischung von 5 ml Trimethylaluminium (2M in Toluol) und 5 ml Toluol wird bei -15° C langsam mit einer Lösung aus 0,435 g Dimethylamin und 5 ml Toluol versetzt. Man lässt die Temperatur während 1 Stunde auf Raumtemperatur steigen und versetzt mit einer Lösung von 1,79 g B4 in 5 ml Toluol. Das Reaktionsgemisch wird noch 22 Stunden bei 80° C nachgerührt. Die Reaktionsmischung wird auf Raumtemperatur gekühlt und langsam mit 20 ml 0,5 N Salzsäure versetzt (exotherme Reaktion). Das Gemisch wird 3 x mit 30 ml Toluol extrahiert und die organischen Phasen nacheinander mit 2 x 30 ml Wasser und 30 ml gesättigter wässriger Natriumhydrogensulfatlösung gewaschen. Die vereingten organischen Phasen werden mit Magnesiumsulfat getrocknet und am Rotavapor eingedampft. Aus dem Rückstand wird mittels Flashchromatographie (SiO₂ 60F / Essigsäureethylester / Hexan 1:2) die Titelverbindung C1 als farbloses Oel erhalten (1,50 g , 84 %).

## Patentansprüche

1. Verbindungen der Formel Ia in Form des Enantiomeren, 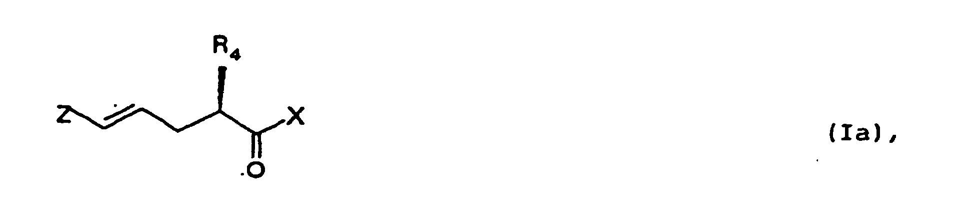 worin
R₄ für C₁-C₆-Alkyl steht, Z Chlor, Brom oder Iod bedeutet, und X -OH, Chlorid, Bromid oder Iodid darstellt, oder X mit dem Carbonylrest eine Estergruppe bildet, sowie Salze der Carbonsäuren.

2. Verbindungen gemäss Anspruch 1, **dadurch gekennzeichnet, dass** R₄ für C₁-C₄-Alkyl steht.

3. Verbindungen gemäss Anspruch 1, **dadurch gekennzeichnet, dass** R₄ für Methyl, Ethyl, n- und i-Propyl, n-, i- und t-Butyl steht.

4. Verbindungen gemäss Anspruch 1, **dadurch gekennzeichnet, dass** R₄ i-Propyl darstellt.

5. Verbindungen gemäss Anspruch 1, **dadurch gekennzeichnet, dass** Z für Cl steht.

6. Verbindungen gemäss Anspruch 1, **dadurch gekennzeichnet, dass** X einen Rest der Formel R₇O- darstellt, worin R₇ eine gegebenenfalls Heteroatome, ausgewählt aus der Gruppe O und N, enthaltende organische Gruppe mit 1 bis 18 C-Atomen bedeutet.

7. Verbindungen gemäss Anspruch 6, **dadurch gekennzeichnet, dass** R₇ C₁-C₄-Alkyl darstellt

8. Verbindungen gemäss Anspruch 1, **dadurch gekennzeichnet, dass** X für Chlorid oder Bromid steht.

9. Verbindungen gemäss Anspruch 1, **dadurch gekennzeichnet, dass** es sich bei den Salzen der Carbonsäuren um Alkalimetall- oder Erdalkalimetallsalze, sowie um Ammoniumsalze handelt.

10. Verbindungen gemäss Anspruch 1, **dadurch gekennzeichnet, dass** Z Chlor bedeutet, R₄ C₁-C₄-Alkyl darstellt, und X für OH, Cl, Br oder C₁-C₄-Alkoxy steht.

11. Verbindungen gemäss Anspruch 10, **dadurch gekennzeichnet, dass** R₄ i-Propyl bedeutet.

12. Verbindungen gemäss Anspruch 1, **dadurch gekennzeichnet, dass** Z Chlor bedeutet, R₄ i-Propyl darstellt, und X für OH, Cl, Br, Methoxy oder Ethoxy steht.

13. Verbindungen gemäss Anspruch 12, **dadurch gekennzeichnet, dass** Z Chlor bedeutet, R₄ i-Propyl darstellt, und X für Cl oder Br steht.

14. Verfahren zur Herstellung von Verbindungen der Formel Ia, **dadurch gekennzeichnet, dass** man eine Verbindung der Formel IV, 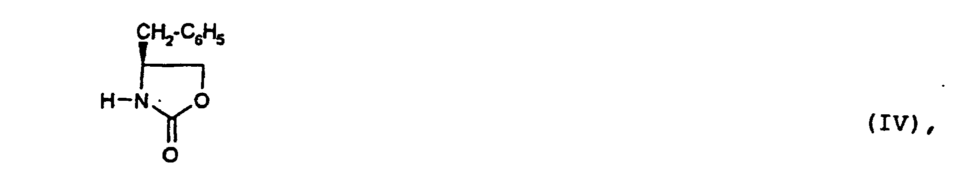 mit einem Carbonsäurehalogenid der Formel R₄CH₂-CO-X umsetzt, worin R₄ die in Anspruch 1 angegebene Bedeutung hat und X für Chlor, Brom oder Iod steht, die erhaltene Verbindung der Formel V, 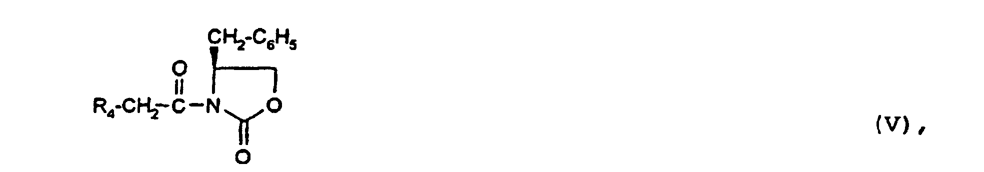 zuerst mit Lithiumhexamethyldisilazid und dann mit einer Verbindung der Formel II umsetzt, 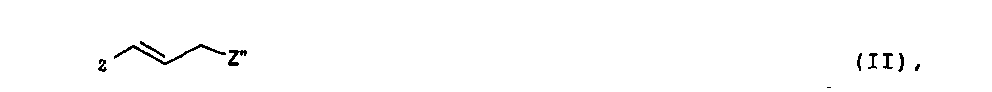 worin Z und Z" unabhängig voneinander Chlor, Brom oder Iod darstellen, danach die erhaltene Verbindung der Formel VI 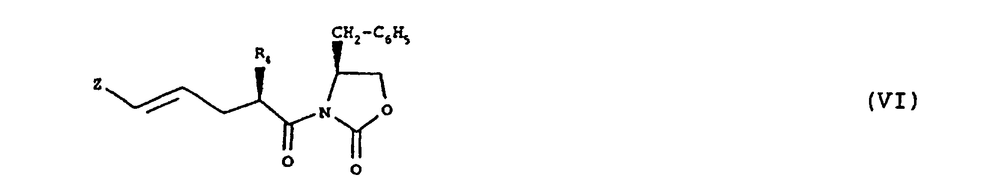 mit einer Base hydrolysiert, die Salze oder Carbonsäuren der Formel Ia isoliert, und gegebenenfalls die Carbonsäuren zu Estern oder Halogeniden derivatisiert.

## Claims

1. Compounds of formula la in the form of the enantiomer, 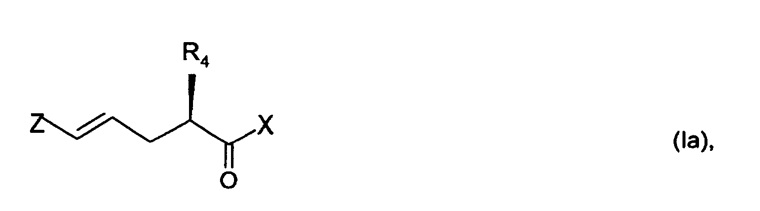 wherein
R₄ is C₁-C₆alkyl, Z is chlorine, bromine or iodine, and X is -OH, chloride, bromide or iodide, or X forms an ester group with the carbonyl substituent, as well as salts of carboxylic acids.

2. Compounds according to claim 1, comprising an embodiment wherein R₄ is C₁-C₄alkyl.

3. Compounds according to claim 1, comprising an embodiment wherein R₄ is methyl, ethyl, n- and i-propyl, n-, i- and t-butyl.

4. Compounds according to claim 1, comprising an embodiment wherein R₄ is i-propyl.

5. Compounds according to claim 1, comprising an embodiment wherein Z is Cl.

6. Compounds according to claim 1, comprising an embodiment wherein X is a substituent of formula R₇O-, wherein R₇ is an organic group with 1 to 18 C-atoms and optionally comprises heteroatoms selected from group O and N.

7. Compounds according to claim 6, comprising an embodiment wherein R₇ is C₁-C₄alkyl

8. Compounds according to claim 1, comprising an embodiment wherein X is chloride or bromide.

9. Compounds according to claim 1, comprising an embodiment wherein the salts of the carboxylic acids are alkali metal or alkaline earth metal salts and ammonium salts.

10. Compounds according to claim 1, comprising an embodiment wherein Z is chlorine, R₄ is C₁-C₄alkyl, and X is OH, Cl, Br or C₁-C₄alkoxy.

11. Compounds according to claim 10, comprising an embodiment wherein R₄ is i-propyl.

12. Compounds according to claim 1, comprising an embodiment wherein Z is chlorine, R₄ is i-propyl, and X is OH, Cl, Br, methoxy or ethoxy.

13. Compounds according to claim 12, comprising an embodiment wherein Z is chlorine, R₄ is i-propyl, and X is Cl or Br.

14. Process for the preparation of compounds of formula la comprising the reaction of a compound of formula IV, 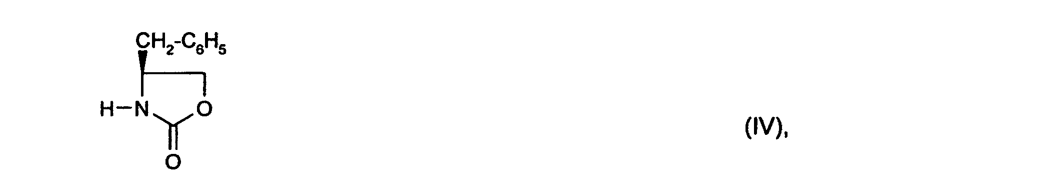 with a carboxylic acid halide of formula R₄CH₂-CO-X, wherein R₄ is as defined in claim 1 and X is chlorine, bromine or iodine, and reaction of the resulting compound of formula V, 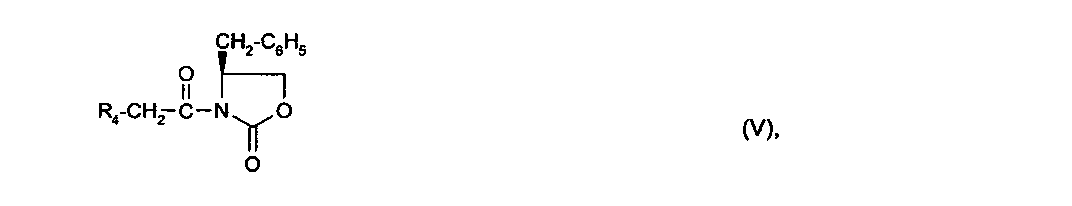 first with lithium hexamethyldisitazide and then with a compound of formula II, 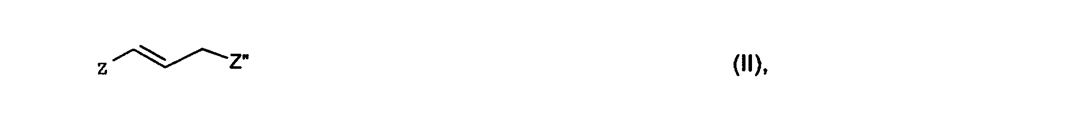 wherein Z and Z" are independently of one another chlorine, bromine or iodine, followed by hydrolyzation of the resulting compound of formula VI 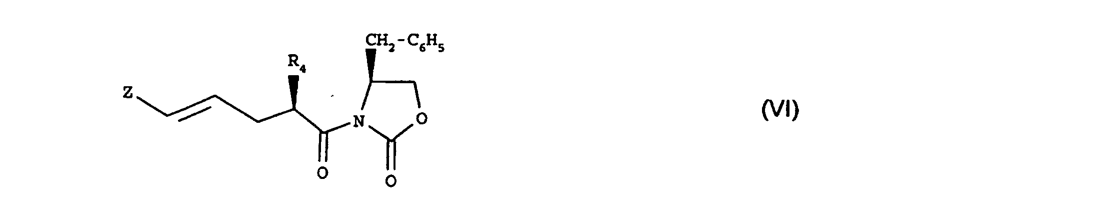 with a base, isolation of the salts or carboxylic acids of formula la, and if necessary derivatization of the carboxylic acids to form esters or halides.

## Revendications

1. Composés de formule Ia sous la forme de l'énantiomère, 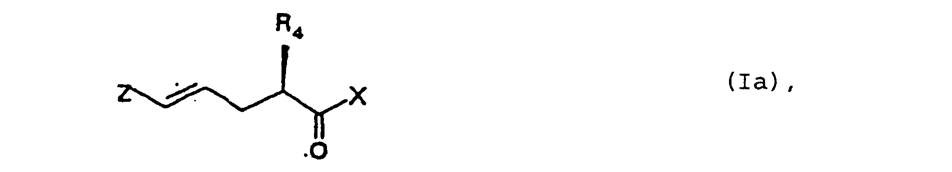 dans lequel
R₄ représente un alkyle en C₁-C₆, Z représente le chlore, le brome ou l'iode, et X représente -OH, un chlorure, bromure ou iodure, ou X forme avec le groupe carbonyle un groupe ester, ainsi que les sels des acides carboxyliques.

2. Composés selon la revendication 1, **caractérisés en ce que** R₄ représente un alkyle en C₁-C₄.

3. Composés selon la revendication 1, **caractérisés en ce que** R₄ représente un méthyle, éthyle, n- et i-propyle, n-, i- et t-butyle.

4. Composés selon la revendication 1, **caractérisés en ce que** R₄ représente un i-propyle.

5. Composés selon la revendication 1, **caractérisés en ce que** Z représente Cl.

6. Composés selon la revendication 1, **caractérisés en ce que** X représente un groupe de formule R₇O-, dans laquelle R₇ représente un groupe organique avec de 1 à 18 atomes de carbone contenant éventuellement des hétéroatomes, choisis dans le groupe O et N.

7. Composés selon la revendication 6, **caractérisés en ce que** R₇ représente un alkyle en C₁-C₄.

8. Composés selon la revendication 1, **caractérisés en ce que** X représente un chlorure ou bromure.

9. Composés selon la revendication 1, **caractérisés en ce qu'**il s'agit pour les sels des acides carboxyliques de sels de métaux alcalins ou de métaux alcalino-terreux, ainsi que de sels d'ammonium.

10. Composés selon la revendication 1, **caractérisés en ce que** Z représente le chlore, R₄ un alkyle en C₁-C₄, et X représente OH, Cl, Br ou un alcoxy en C₁-C₄.

11. Composés selon la revendication 10, **caractérisés en ce que** R₄ représente un i-propyle.

12. Composés selon la revendication 1, **caractérisés en ce que** Z représente le chlore, R₄ un i-propyle, et X représente OH, Cl, Br un méthoxy ou éthoxy.

13. Composés selon la revendication 12, **caractérisés en ce que** Z représente le chlore, R₄ un i-propyle, et X représente Cl ou Br.

14. Procédé pour la préparation de composés de formule Ia, **caractérisé en ce qu'**on met à réagir un composé de formule IV, 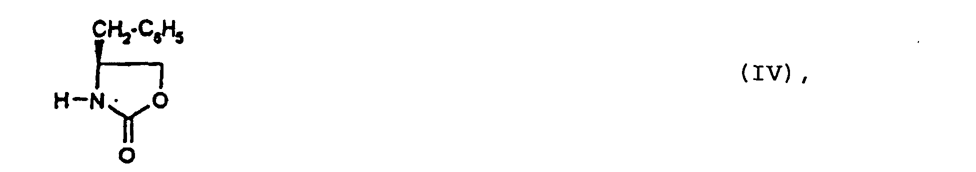 avec un halogénure d'acide carboxylique de formule R₄CH₂-CO-X, dans laquelle R₄ a la signification indiquée dans la revendication 1 et X représente le chlore, le brome ou l'iode, on met à réagir le composé obtenu de formule V, 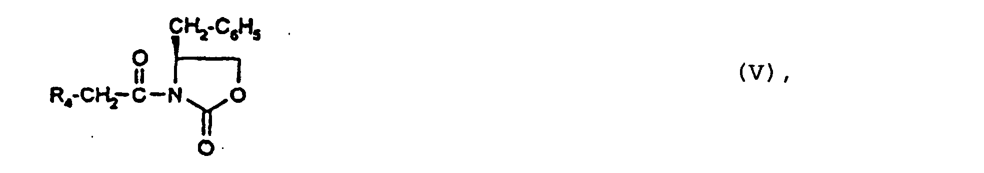 d'abord avec l'hexaméthyldisilazide de lithium et ensuite avec un composé de formule II, 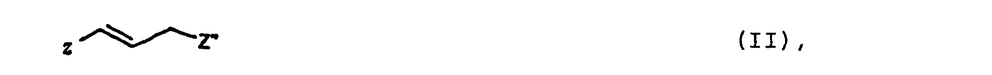 dans laquelle Z et Z'' représentent indépendamment l'un de l'autre le chlore, le brome ou l'iode, ensuite on hydrolyse le composé obtenu de formule VI 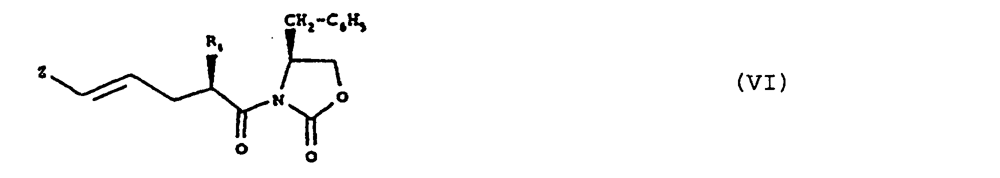 avec une base, on isole les sels ou les acides carboxyliques de formule Ia, et on transforme éventuellement les acides carboxyliques en dérivés esters ou halogénures.
